# EUROPEAN PATENT SPECIFICATION

(11) **EP 1 463 065 B1**
(45) Date of publication and mention of the grant of the patent: **17.12.2008**
(21) Application number: 02775219.5
(22) Date of filing: 20.09.2002
(51) Int. Cl.: A61K 51/02, G21G 1/10

(54) **PROCESSES FOR PRODUCING 15O-CARBON MONOXIDE AND 15O-CARBON DIOXIDE AND PRODUCTION APPARATUS**
PROZESSE ZUR HERSTELLUNG VON 15O-KOHLENMONOXID UND 15O-KOHLENDIOXID UND HERSTELLUNGSVORRICHTUNG
PROCEDES DE PRODUCTION DE MONOXYDE DE 15O-CARBONE ET DE DIOXYDE DE 15O-CARBONE ET APPAREIL DE PRODUCTION

(30) Priority: 29.11.2001 JP 2001364011
(43) Date of publication of application: 29.09.2004
(73) Proprietor: IIDA, Hidehiro, Minoo-shi Osaka 562-0004 (JP)
(72) Inventor: IIDA, Hidehiro, Osaka 562-0004 (JP); MIYAKE, Yoshinori, Osaka 585-0845 (JP)
(74) Representative: Bassil, Nicholas Charles
(86) International application number: PCT/JP2002/009712
(87) International publication number: WO 2003/046930

(56) References cited:
- JP-A- 9 113 693
- JP-A- 9 113 694
- PATENT ABSTRACTS OF JAPAN vol. 013, no. 084 (C-572), 27 February 1989 (1989-02-27) & JP 63 270305 A (SUMITOMO HEAVY IND LTD), 8 November 1988 (1988-11-08)
- TOCHON-DANGUY H J ET AL: "Positron emission tomography: radioisotope and radiopharmaceutical production." AUSTRALASIAN PHYSICAL & ENGINEERING SCIENCES IN MEDICINE / SUPPORTED BY THE AUSTRALASIAN COLLEGE OF PHYSICAL SCIENTISTS IN MEDICINE AND THE AUSTRALASIAN ASSOCIATION OF PHYSICAL SCIENCES IN MEDICINE, (1999 DEC) 22 (4) 136-44. REF: 34 JOURNAL CODE: 82, December 1999 (1999-12), XP008058731
- HELUS, F. ET AL: "Remotely controlled on-line production of different species labeled with oxygen-15" JOURNAL OF RADIOANALYTICAL AND NUCLEAR CHEMISTRY , 165(5), 327-30 CODEN: JRNCDM; ISSN: 0236-5731, 1992, XP008058713
- STRIJCKMANS, K. ET AL: "Production and quality control of oxygen(15O2) and carbon dioxide - 15O2 for medical use" INTERNATIONAL JOURNAL OF APPLIED RADIATION AND ISOTOPES , 36(4), 279-83 CODEN: IJARAY; ISSN: 0020-708X, 1985, XP008058923
- MACKAY D.B. ET AL.: 'Quality assurance for PET gas production using the cyclone 3D oxygen-15 generator' APPLIED RADIATION AND ISOTOPES vol. 51, no. 4, 1999, pages 403 - 409, XP004172292
- LOADER N.J. ET AL.: "Rapid catalytic oxidation of CO to CO2 - On the development of a new approach to on-line oxygen isotope analysis of organic matter" RAPID COMMUNICATION IN MASS SPCTROMETRY, vol. 13, 1999, pages 1828-1832,

## Description

### Technical Field

The present invention relates to ¹⁵O-carbon monoxide and ¹⁵O-carbon dioxide, which are useful in positron emission tomography (PET). ¹⁵O-carbon monoxide and ¹⁵O-carbon dioxide are used to measure the level of oxygen metabolism, important information for identifying ischemic conditions in brain tissue.

Hereinafter, ¹⁵O-carbon monoxide is represented by C¹⁵O, and ¹⁵O-carbon dioxide is represented by C¹⁵O₂. Furthermore, carbon monoxide comprising C¹⁵O is represented by [¹⁵O]CO, and carbon dioxide comprising C¹⁵O₂ is represented by [¹⁵O]CO₂.

### Background Art

Positrons (positive electrons) are antiparticles comprising a charge opposite to that of electrons. On colliding with electrons, positrons discharge γ-rays and then disappear (β⁺ decay). Elements comprising positrons in their nucleus are called positron nuclides. Positron nuclides can be obtained by irradiating light elements with proton or deuteron beams to produce elements in which the number of protons is larger than the number of neutrons. ¹¹C, ¹³N, ¹⁵O, ¹⁸F, and such are known as positron nuclides. For example, while the number of neutrons in normal element ¹⁶O is eight, the number of neutrons in positron nuclide ¹⁵O is seven, less than the number of its protons, which is eight. Therefore, ¹⁵O discharges a positron to reduce its protons to seven, resulting in a stable nitrogen element.

The half-life of positron nuclides is very short: about 20 minutes for ¹¹C, and 118-130 seconds for ¹⁵O. Since positron nuclide radioactivity decreases rapidly, synthesis of tracer labels and kinetics measurement and such are time-constrained. However, since positron nuclides comprise the following advantages, they are applied in the field of nuclear medicine in diagnosing living organisms. The diagnostic method utilizing positron nuclides is called positron emission tomography (PET).
(1) These positron nuclides are isotopes of elements that make up molecules originally present in living organisms. Therefore, by utilizing positron nuclides as tracers, the metabolic condition of living organisms can be observed without modifying molecules present in organisms.
(2) Due to their short half-life, exposure to living organisms is low (repeated tests and challenge tests can be carried out).
(3) Due to their short half-life, specific radioactivity is high, and thus diagnosis using trace amounts of nuclide is possible.

In PET, the location of a tracer in a three-dimensional space in a living organism is converted into an image, based on the theory below. As described above, positrons collide with electrons and discharge γ-rays. Specifically, two annihilation photons (γ-rays) with an energy of 511 keV are emitted in opposite directions, at 180° to each other. The emitted γ-rays are measured using radiation detectors placed in a straight line. When counted by a coincidence circuit, nuclear decay is found to occur along this straight line. Furthermore, when these detectors are placed in a 360° circle, the distribution of positron nuclides can be tomographically imaged based on approximately the same theory as X-ray CT. Additionally, the amount of detected radiation can be quantitatively determined.

In PET, the concentration of positron nuclides inside the body is accumulated as tomographic images. Consequently, if a region of interest (ROI) is set in an arbitrary portion, the concentration of positron nuclides at this position can also be determined. Furthermore, when a tracer's metabolic pathway is already known, and a kinetics analysis model in the body has been established, the tracer's behavior in the body can be quantitatively understood. By utilizing such PET features, the metabolic condition of various organs can be non-invasively observed, from outside of the body. As the diagnostic methods using PET, for example, the following diagnoses are in practical use:

| Tracer | Diagnostic target |
|---|---|
| ¹⁵O gas | Local cerebral oxygen metabolism |
| [¹⁵O]CO gas | Local cerebral blood volume |
| [¹⁵O]CO₂ gas | Local cerebral blood flow |
| ¹⁵O-H₂O | Local cerebral blood flow |
| ¹⁸F-DG | Glucose metabolism |
| ¹⁸F-DOPA | Dopamine-based neural transmission function |

For example, in cerebral stroke therapy, appropriate treatment within three hours of onset is said to significantly influence the subsequent medical condition. Thus, rapid and accurate understanding of post-onset cerebral oxygen metabolism is important for treatment. At present, PET examinations are the only examinations capable of correctly understanding cerebral oxygen metabolism.

PET examination of cerebral oxygen metabolism is conducted as described below. First, a test subject inhales the following three gases as tracers: [¹⁵O]CO gas; [¹⁵O]CO₂ gas; and ¹⁵O gas.

After inhalation, tracer distribution in the subject's head is traced using PET. Local cerebral blood volume, local cerebral blood flow, and local cerebral oxygen uptake ratio can be measured. Based on the results of these measurements, the degree of local cerebral oxygen metabolism is calculated.

Inhalation of the three above-mentioned ¹⁵O-labeled gases is necessary for PET examination of cerebral oxygen metabolism. Due to the preparation of each gas and photography, examination takes about two hours. The applicable scope of this examination is significantly limited, since examination of patients seeking treatment within three hours of onset requires two or more hours. In fact, the value of examination results is increased by initiating examination as soon as possible after onset. Therefore, technology that enables examination of cerebral oxygen metabolism in a shorter time is required.

Enhanced acute-stage medical care for cerebral stroke is necessary in an aging society. Test methods able to be performed within 30 minutes have been developed as test methods for cerebral stroke, which requires rapid examination. For example, in about five to 30 minutes, diagnostic imaging of the brain by X-ray computed tomography (CT) and magnetic resonance imaging (MRI) can be performed. These examinations are effective for diagnosing cerebrovascular disorders such as hemorrhage and infarction. However, when using X-ray CT and MRI it is difficult to understand the brain tissue dysfunctions caused by to these cerebrovascular disorders. To confirm the condition of brain tissue, examination of local cerebral oxygen metabolism is necessary. Therefore, even after the practical application of these examination methods, which can be performed in a short time, examination of cerebral oxygen metabolism using ¹⁵O as a tracer is important.

In general, a ¹⁵O gas is prepared by the following process. First, nitrogen gas comprising 0.5% to 2.5% oxygen gas is irradiated with deuterons. ¹⁵O gas is then generated by the nuclear reaction of ¹⁴N(d,n)¹⁵O. Deuterons are irradiated using a cyclotron. ¹⁵O gas thus-produced is itself utilized as an inhalation gas for examining cerebral oxygen metabolism using the above-mentioned PET. In addition, it can also be utilized in the preparation of [¹⁵O]CO gases. A [¹⁵O]CO gas can be prepared by reacting the recovered ¹⁵O gas with activated carbon heated to 1000°C.

[¹⁵O]CO₂ gas required for PET examination of cerebral oxygen metabolism is also prepared as described below. First, nitrogen gas comprising 0.25% to 2.5% oxygen gas is irradiated with deuterons. ¹⁵O gas is then generated by the nuclear reaction of ¹⁴N(d,n)¹⁵O. A [¹⁵O]CO₂ gas can be prepared by reacting the recovered ¹⁵O gas with activated carbon heated to 400°C.

In addition, a method is also known for directly synthesizing a [¹⁵O]CO₂ gas, and the target gas in this method is nitrogen gas comprising carbon dioxide (Mackay DB, Quality assurance for PET gas production using the Cyclone 3D oxygen-15 generator. Applied Radiation and Isotopes, 51, 1999, 403-409). In this method, carbon dioxide synthesis does not require a heating process. However, an activated carbon column heated to 500°C is required when removing carbon monoxide generated by deuteron beam irradiation. Thus, this method also necessitates a heating process.

As described above, known methods for preparing [¹⁵O]CO and [¹⁵O]CO₂ require a step of contacting the gas with activated carbon, a catalyst, or an adsorbent under conditions of high temperature. In general, high temperature conditions are created by heating an electric furnace. However, heating an electric furnace requires considerable time, precluding any reduction in the duration of the tracer gas supply process. If [¹⁵O]CO and [¹⁵O]CO₂ can be produced at lower temperatures, tracer gases can be produced in a shorter period of time. If tracer gases can be supplied in a shorter period of time, PET examination of cerebral oxygen metabolism can be completed in a shorter time. By early diagnosis, more efficient methods of cerebral stroke treatment can be selected. Furthermore, by reducing examination time, necessary examinations can be performed on a larger number of patients.

### Disclosure of the Invention

An objective of the present invention is to provide methods for preparing [¹⁵O]CO and [¹⁵O]CO₂ in a short period of time. More specifically, an objective of the present invention is to provide methods for preparing [¹⁵O]CO and [¹⁵O]CO₂ that do not require conditions of high temperature.

As a result of various investigations to achieve this objective, the present inventors discovered that [¹⁵O]CO is continuously generated immediately after a target gas is irradiated with deuterons. Furthermore, the present inventors also discovered that [¹⁵O]CO can be rapidly converted into ¹⁵O-carbon dioxide at room temperature by using an oxidizing agent. The present inventors have found that the length of a PET examination of cerebral oxygen metabolism can be significantly reduced by utilizing the [¹⁵O]CO and [¹⁵O]CO₂ obtained by these methods, thus completing the present invention.

Namely, the present invention relates to methods for producing [¹⁵O]CO or [¹⁵O]CO₂, and apparatuses for the same.
[1] A method for producing ¹⁵O-carbon monoxide, which comprises the steps of:
   (1) using a nitrogen gas comprising carbon monoxide as a gas to be irradiated, and irradiating the gas with a deuteron beam; and
   (2) recovering ¹⁵O-carbon monoxide produced by the deuteron beam irradiation.
[2] The method according to [1], wherein the amount of carbon monoxide comprised in the gas to be irradiated is 0.01 to 0.5 v/v%.
[3] The method according to [2], wherein the amount of carbon monoxide comprised in the gas to be irradiated is 0.05 to 0.2 v/v%.
[4] The method according to [1], which comprises the step of contacting the irradiated gas with an active carbon column and/or a soda lime column, and recovering ¹⁵O-carbon monoxide.
[5] The method according to [1], wherein the gas to be irradiated is continuously supplied while recovering the produced ¹⁵O-carbon monoxide.
[6] An apparatus for producing ¹⁵O-carbon monoxide, which comprises:
   (1) a target box through which a gas to be irradiated can be passed and in which the gas to be irradiated can be irradiated with a deuteron beam;
   (2) a means of supplying the gas to be irradiated, which supplies the target box of (1) with a nitrogen gas comprising carbon monoxide as the gas to be irradiated; and
   (3) a means for recovering ¹⁵O-carbon monoxide.
[7] The apparatus according to [6], wherein the means of (3) for recovering ¹⁵O-carbon monoxide comprises:
   a means for receiving the gas to be irradiated in the target box of (1); and
   an active carbon column and/or a soda lime column.
[8] The apparatus according to [6], wherein the means for supplying the gas to be irradiated of (2) can continuously supply the target box of (1) with the gas to be irradiated.
[9] The apparatus according to [6], wherein the means of (3) for recovering ¹⁵O-carbon monoxide is connected to a line for supplying ¹⁵O-carbon monoxide to a subject.
[10] A method for producing ¹⁵O-carbon dioxide, which comprises the steps of:
   (1) contacting ¹⁵O-carbon monoxide with a manganese dioxide-copper oxide (II) catalyst in the presence of dry oxygen; and
   (2) recovering the produced ¹⁵O-carbon dioxide.
[11] The method according to [10], wherein the dry oxygen is a mixed gas comprising dry carbon dioxide.
[12] The method according to [10] for producing ¹⁵O-carbon monoxide, which comprises the steps of:
   i) supplying the target box with a nitrogen gas comprising carbon monoxide as the gas to be irradiated; and
   ii) irradiating the gas to be irradiated with a deuteron beam to produce ¹⁵O-carbon monoxide.
[13] An apparatus for producing ¹⁵O-carbon dioxide, which comprises:
   (1) a means for supplying ¹⁵O-carbon monoxide;
   (2) a manganese dioxide-copper oxide (II) catalyst for oxidizing carbon monoxide;
   (3) a means for supplying dry oxygen to a manganese dioxide-copper oxide (II) catalyst; and
   (4) a means for recovering ¹⁵O-carbon dioxide.
[14] The apparatus according to [13], wherein the means of (1) for supplying ¹⁵O-carbon monoxide is a ¹⁵O-carbon monoxide-producing apparatus that comprises:
   i) a target box through which a gas to be irradiated can be passed, and in which the gas to be irradiated can be irradiated with a deuteron beam; and
   ii) a means for supplying the gas to be irradiated which supplies the target box of (i) with a nitrogen gas comprising carbon monoxide as the gas to be irradiated.
[15] A method for producing ¹⁵O-carbon monoxide and 150-carbon dioxide, which comprises the steps of:
   (1) using a nitrogen gas comprising carbon monoxide as a gas to be irradiated, and irradiating the gas with a deuteron beam;
   (2) recovering the ¹⁵O-carbon monoxide produced by the deuteron beam irradiation;
   (3) contacting the irradiated gas with a manganese dioxide-copper oxide (II) catalyst in the presence of dry oxygen; and
   (4) recovering ¹⁵O-carbon dioxide produced in the step of (3).
[16] An apparatus for producing ¹⁵O-carbon monoxide and ¹⁵O-carbon dioxide, which comprises:
   (1) a target box through which a gas to be irradiated can be passed, and in which the gas to be irradiated can be irradiated with a deuteron beam;
   (2) a means for supplying the gas to be irradiated, which supplies the target box with a nitrogen gas comprising carbon monoxide as the gas to be irradiated;
   (3) a means for recovering ¹⁵O-carbon monoxide;
   (4) a manganese dioxide-copper oxide (II) catalyst for oxidizing carbon monoxide;
   (5) a means for supplying dry oxygen to a manganese dioxide-copper oxide (II) catalyst; and
   (6) a means for recovering ¹⁵O-carbon dioxide.

Furthermore, the present invention provides methods for examining cerebral blood flow, which comprise the steps of:
(1) administering a subject with [¹⁵O]CO produced by the method of [1], [¹⁵O]CO₂ produced by the method of [10], and ¹⁵O;
(2) detecting the distribution of positron nuclide ¹⁵O in the subject's brain; and
(3) based on the distribution of positron nuclide ¹⁵O, determining at least one parameter selected from the group consisting of local cerebral blood volume, local cerebral blood flow, and local cerebral oxygen uptake ratio.

Furthermore, the present invention provides methods for examining the degree of local cerebral oxygen metabolism, based on the above-mentioned examination methods. Namely, the present invention relates to methods for examining the degree of local cerebral oxygen metabolism, which comprise the steps of:
(1) using an above-mentioned cerebral blood flow examination method to determine local cerebral blood volume, local cerebral blood flow, and local cerebral oxygen uptake ratio; and
(2) determining the degree of local cerebral oxygen metabolism based on the parameters determined in (1).

The present invention relates to methods for producing [¹⁵O] CO, which comprise the steps of:
(1) using a nitrogen gas comprising carbon monoxide as the gas to be irradiated, and irradiating the gas to be irradiated with a deuteron beam;
(2) recovering [¹⁵O] CO produced by the deuteron beam irradiation.

In the present invention, a "gas to be irradiated" means a gas to which a carrier gas is added. In general, "target gas" means a gas comprising, in a gaseous state, elements that produce positron nuclides by nuclear reaction. In the present invention, the target gas can be high purity nitrogen gas which comprises a small amount of carbon monoxide as a carrier. A high purity gas is used as the target gas (nitrogen gas). ¹⁴N is generally used as the nitrogen gas. ¹⁵N can also be used to obtain positron nuclides by ¹⁵N(p,n)¹⁵O reaction, but is expensive. Therefore, a preferable target gas is high purity nitrogen gas (¹⁴N). In the present invention, "high purity gas" denotes a gas of 99.9999 purity or more.

The gas to be irradiated of the present invention comprises carbon monoxide gas as a carrier. Carbon monoxide gas is added for the recovery of [¹⁵O]CO in the gas irradiated with deuteron beams. The percentage of carbon monoxide gas in the gas to be irradiated is usually 0.5% or less. For example, depending on the concentration of carbon monoxide gas, when [¹⁵O]CO₂ is synthesized using oxidizing catalysts as described below, non-radioactive by-products may be produced since oxidizing catalysts increase temperature. Therefore, it is usually preferable that the concentration of carbon monoxide gas is 0.5% or less. Furthermore, the [¹⁵O]CO inhaled by a subject should be of a concentration that does not risk carbon monoxide poisoning. In general, a safe range for carbon monoxide poisoning is said to be less than 1%. Consequently, 0.5% or less carbon monoxide gas is also preferable from the standpoint of safety. More specifically, 0.05 to 0.1% v/v of carbon monoxide gas is preferable.

In the present invention, the gas to be irradiated is irradiated with deuteron beams. In general, the gas to be irradiated is irradiated by passing deuteron beams through a part of its flow pass. Preferably, the part of the flow pass of the gas to be irradiated through which deuteron beams pass comprises a structure able to store a certain amount of the gas to be irradiated. In the present invention, the structure capable of storing the gas to be irradiated is called a "target box". The target box constitutes a part of the flow pass of the gas to be irradiated, and can be a space with a large volume that partially constitutes the flow pass. Furthermore, to store the gas to be irradiated, the target box can also comprise a mechanism for controlling the flow of the gas to be irradiated. The target box has some length in the traveling direction of the deuteron beams, allowing effective irradiation of the gas to be irradiated. The material of the target box is not particularly limited, as long as it allows permeation of deuteron beams and hermetically seals the gas to be irradiated. In general, an aluminum-magnesium alloy is used as the target box material.

When the gas to be irradiated is irradiated with deuteron beams under suitable conditions, [¹⁵O]CO is continuously generated from a target gas, nitrogen, immediately after irradiation. In this case, [¹⁵O]CO is considered to be generated by the recoil energy of ¹⁴N(d,n)¹⁵O reaction products. For example, when the above-mentioned target gas is irradiated with 6-10 MeV deuterons, [¹⁵O]CO can be produced continuously, immediately after deuteron irradiation.

[¹⁵O]CO generated by deuteron beam irradiation can be made to flow out of the target box by continually supplying new gas to be irradiated to the target box. C¹⁵O produced by a method of the present invention can be obtained as a gas mixture with a carrier gas by recovering radiated gas from the gas that has flowed out. Furthermore, impurities can also be removed as necessary. When a nitrogen gas comprising carbon monoxide is used as the gas to be irradiated, the gas may comprise carbon dioxide generated by carbon monoxide oxidation, nitrogen oxide generated by nitrogen oxidation. Since these by-products may impart an erroneous result in PET examination of cerebral oxygen metabolism, they are preferably removed in advance.

Methods for removing these by-products are well known. For example, carbon dioxide can be absorbed and removed by passing through a soda lime column. Nitrogen oxide can be effectively removed by passing through an activated carbon column. The gas to be irradiated from which by-products have been removed can be used as an inhalation gas for PET examination of cerebral oxygen metabolism, after adjusting carbon monoxide concentration, and mixing with other gases, as necessary. The inhalation gas is generally prepared by mixing a large amount of air (20 to 40 L/min) with a gas to be irradiated (300 to 500 mL/min) that comprises [¹⁵O] gas.

The [¹⁵O]CO production methods of the present invention, which lead from supplying the gas to be irradiated to removing by-products, can be performed using well-known positron synthesis apparatus. The present invention relates to [¹⁵O]CO production apparatus comprising:
(a) a target box through which a gas to be irradiated can pass, and in which that gas is irradiated by deuteron beams;
(b) a means for supplying the gas to be irradiated, which supplies the target box of (a) with a nitrogen gas comprising carbon monoxide as the gas to be irradiated; and
(c) a means for recovering [¹⁵O]CO.

The target box of (a) is connected to the means for supplying the gas to be irradiated of (b). The means of (b) supplies the target box of (a) with the above-mentioned target gas comprising a suitable concentration of carbon monoxide. To regulate carbon monoxide concentration and control the amount of the gas to be irradiated supplied to the target box (a), the means for supplying the gas to be irradiated of (b) can comprise a gas flow rate regulation mechanism. The gas flow rate regulation mechanism is made up of, for example, a solenoid valve and a gas flow meter. The carbon monoxide concentration and amount of target gas supplied are regulated by regulating the flow rate of a target gas and carbon monoxide. They can also be managed to maintain a given numerical value by using a computer system that monitors gas flow rate.

The gas to be irradiated that is supplied to the target box of (a) is irradiated with deuteron beams. The target box is made of a material for which irradiation by deuteron beams is possible, and placed at a deuteron beam irradiation position. To efficiently irradiate the gas to be irradiated with deuteron beams, the target box preferably comprises a form capable of aligning the gas to be irradiated in the direction in which the deuteron beams are traveling.

Deuteron beams can be supplied by a cyclotron. Cyclotrons that radiate deuteron beams are well known. A typical cyclotron is made up of an ion source, accelerating electrodes.

Since deuterons comprising an energy of 4-8 MeV cause ¹⁴N(d,n)¹⁵O nuclear reactions to generate ¹⁵O compounds, a cyclotron accelerator with an acceleration capability in this energy range is suitable for medical applications. The charged particles used in the nuclear reactions are all positive, however, there are positive charge acceleration-types and negative charge-types, depending on differences in the charge of the particles while accelerating.

The means of (c) for recovering [¹⁵O]CO is a means for recovering a gas to be irradiated which has been irradiated with deuterons in the target box of (a). The means of (c) for recovering [¹⁵O]CO can be, for example, a gas flow pass connected to the target box of (a). The means of (c) for recovering [¹⁵O]CO can comprise a flow rate controller capable of controlling the flow rate of a gas to be irradiated that flows from the target box of (a). The flow rate controller monitors the flow rate of the gas to be irradiated that is flowing in a flow pass, and controls solenoid valves provided at respective positions according to the production processes, to totally control the amount of the gas to be irradiated flowing in the apparatus of the present invention. For example, when irradiating with deuterons, the amount of the gas to be irradiated that passes through the target box of (a) is controlled so as to store the gas to be irradiated in the target box of (a), in order to irradiate the necessary dose. When recovering the irradiated gas, new gas to be irradiated is fed into the target box of (a), and the irradiated gas flows out of the target box of (a) and is recovered downstream of the target box of (a). The gas to be irradiated can be continuously supplied, and deuteron beam irradiation and recovery of the irradiated gas can also be carried out continuously.

In the [¹⁵O]CO production apparatus of the present invention, the means of (c) for recovering [¹⁵O]CO can comprise a means for removing by-products comprised in the gas to be irradiated. Specifically, carbon dioxide can be absorbed and removed by passing through a soda lime column. Nitrogen oxide can be removed effectively by passing through an activated carbon column. Since these by-products can be removed at normal temperatures, heating is not required.

The above-mentioned means of (c) for recovering [¹⁵O] CO can also be connected to another gas flow pass. For example, it can be directly connected to an inhalation apparatus for enabling a PET subject to inhale a tracer gas. The connection of the means of (c) to an inhalation apparatus is preferable since it enables tracer gases with short half-lives to be rapidly supplied to a subject. Alternatively, the means of (c) can also be connected to a line for producing carbon dioxide, as described later. In the case of connection to a line for the production of carbon dioxide, the gas to be irradiated need not pass through a soda lime column.

Next, the present invention relates to methods of producing [¹⁵O]CO₂, which comprise the steps of:
(1) contacting [¹⁵O]CO with a manganese dioxide-copper oxide (II) catalyst in the presence of dry oxygen; and
(2) recovering the [¹⁵O]CO₂ which is produced.

Specifically, oxidizing catalysts in the present invention comprise a manganese dioxide-copper oxide (II) catalyst. A reaction in which the manganese dioxide-copper oxide (II) catalyst oxidizes carbon monoxide in the presence of oxygen to produce carbon dioxide is well known. However, this kind of catalyst has not been found useful for producing, the [¹⁵O] CO₂ necessary for PET examination of cerebral oxygen metabolism.

In the present invention, commercially available manganese dioxide-copper oxide (II) catalysts can be used as they are. For example, a 150 mm long glass tube of φ 12 mm outer diameter is filled with 2 g of a manganese dioxide-copper oxide (II) catalyst and dried by heating at 120°C for two hours. After cooling, the manganese dioxide-copper oxide (II) catalyst is contacted with carbon monoxide gas in the presence of dry oxygen. The manganese dioxide-copper oxide (II) catalysts that can be utilized in the present invention comprise, for example, CARULITE^{®} Catalyst (manufactured by STREM Chemicals, INC (Newburyport, MA, USA)). As described above, the concentration of carbon monoxide gas is 0.5% or less, and oxygen is supplied in an amount necessary for an oxidation reaction.

Carbon dioxide can also be added to oxygen as a carrier for C¹⁵O₂. Non-radioactive carbon dioxide added to dry oxygen acts as a carrier for C¹⁵O₂ produced by oxidizing catalysts, and contributes to enhancement of the radiochemical purity of C¹⁵O₂ in the final product. The amount of carbon dioxide added as a carrier may be suitably selected depending on the mixing ratio of oxygen to carbon dioxide, and the flow rate of the mixed gas. For example, the above-mentioned non-radioactive carbon dioxide can be added to the mixed gas so as to be 2.5 v/v% or less of the [¹⁵O]CO₂ produced as described above. Specifically, suitable oxidation conditions can be obtained by supplying 10 mL/min of oxygen gas for 300 mL/min of the gas to be irradiated. When carbon dioxide is added as a carrier, oxygen gas comprising 50 v/v% carbon dioxide may be supplied at 10 mL/min.

In the production of [¹⁵O]CO₂ of the present invention, the methods for producing [¹⁵O]CO are not limited. The objective of the present invention is to provide methods for [¹⁵O]CO₂ production that do not require a heating process. Therefore, ideally, [¹⁵O]CO to be utilized as a raw material can also be produced by methods that do not require a heating process. Accordingly, the above-mentioned [¹⁵O]CO production methods are preferable as methods for supplying raw material for the [¹⁵O]CO₂ production methods of the present invention.

In the [¹⁵O]CO₂ production methods of the present invention, [¹⁵O]CO is contacted with a manganese dioxide-copper oxide (II) catalyst in the presence of dry oxygen. In the present invention, a mixed gas of dry oxygen and dry carbon dioxide can also be utilized as dry oxygen.

"Dry oxygen" and "dry carbon dioxide" respectively mean oxygen and carbon dioxide from which water is removed. These dry gases can be obtained by contacting an oxygen gas or carbon dioxide gas with a desiccant such as a silica gel column. Silica gel used as a desiccant is typically dried at 120°C for three hours. Dry oxygen and dry carbon dioxide for use in the present invention can be obtained by using any dessicant, as long as that dessicant has a lower water content than that of a dry gas obtainable using a silica gel column. More specifically, oxygen or carbon dioxide gases with a water content of 2 ppm or less can be used as a dry gas.

Dry oxygen can be used in an amount capable of sufficiently oxidizing [¹⁵O]CO in the presence of an oxidizing catalyst to be used. Therefore, the amount of oxygen added is regulated depending on the nature of the [¹⁵O]CO used as a raw material, and of the oxidizing catalyst. Those skilled in the art can easily set suitable conditions.

When, for example, the above-mentioned manganese dioxide-copper oxide (II) catalyst is used as a catalyst, a side reaction due to heat generation may occur if the concentration of carbon monoxide is high. Therefore, the condition of carbon monoxide concentration may be, for example, 0.5% v/v or less, specifically in the range of 0.05% to 0.3% v/v, preferably 0.07% to 0.2% v/v. The dry oxygen concentration in this case is, for example, 5% v/v or less, typically from 1 to 5% v/v, preferably from 2 to 4% v/v.

When the manganese dioxide-copper oxide (II) catalyst is used, the temperature at which the catalyst is used is not particularly limited. A manganese dioxide-copper oxide (II) catalyst can usually catalyze the oxidation reaction of carbon monoxide at 10-40°C. The reaction can be conducted at any temperature within this temperature range. Therefore, the reaction may be carried out without positive temperature control, or by heating oxidizing catalysts in a range that does not impede the time reduction.

[¹⁵O]CO₂ produced by the action of oxidizing catalysts is recovered and purified, as necessary, and can then be utilized as an inhalation gas for PET examination of cerebral oxygen metabolism. According to the methods for producing [¹⁵O]CO₂ of the present invention, [¹⁵O]CO₂ can be obtained by the oxidation of [¹⁵O]CO as a raw material. Therefore, removal of [¹⁵O]CO is not necessary in the present invention. Since removal of [¹⁵O]CO is not required, a heating process is not required either. Consequently, if [¹⁵O]CO is supplied as a raw material by a method that does not require a heating process, production methods that do not require heating for any process can be realized.

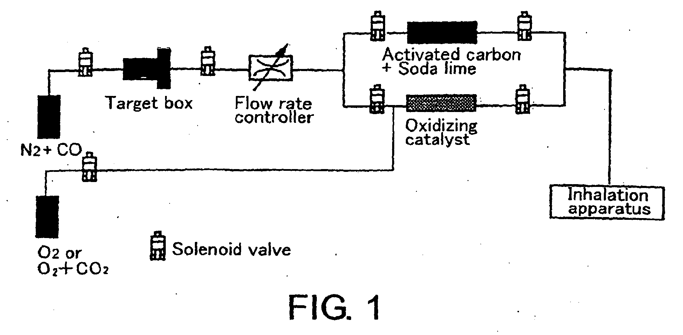

The [¹⁵O]CO₂ production methods based on the present invention can be performed using, for example, the above-described [¹⁵O]CO production apparatus of the present invention. Specifically, a flow pass is set up that branches from the means for recovering the [¹⁵O]CO supplied by the above-mentioned [¹⁵O]CO production apparatus, and mixes dry oxygen gas, or a gas mixture of dry oxygen and dry carbon dioxide gas, and guides the resultant mixture to the oxidizing catalysts. The organization of such a flow pass is shown in Fig. 1. Solenoid valves can be placed before and after an oxidizing catalyst to control the amount of raw material gas supplied to the oxidizing catalysts, and the flow rate of the resulting ¹⁵O-carbon dioxide. Furthermore, a silica gel column for drying oxygen can also be provided in an oxygen-supplying pass.

Similar to above-mentioned [¹⁵O]CO, [¹⁵O]CO₂ produced by the present invention can be supplied directly to an inhalation apparatus, as an inhalation gas for PET examination of cerebral oxygen metabolism.

### Brief Description of the Drawings

Fig. 1 is a diagram showing the organization of an apparatus for supplying [¹⁵O]CO and [¹⁵O]CO₂ according to the present invention.
Fig. 2 is a diagram showing the organization of a system according to the present invention for producing [¹⁵O]CO and [¹⁵O]CO₂, and for measuring their radiochemical purity.

### Best Mode for Carrying out the Invention

Herein below, the present invention is specifically illustrated using Examples.

### [EXAMPLE 1]

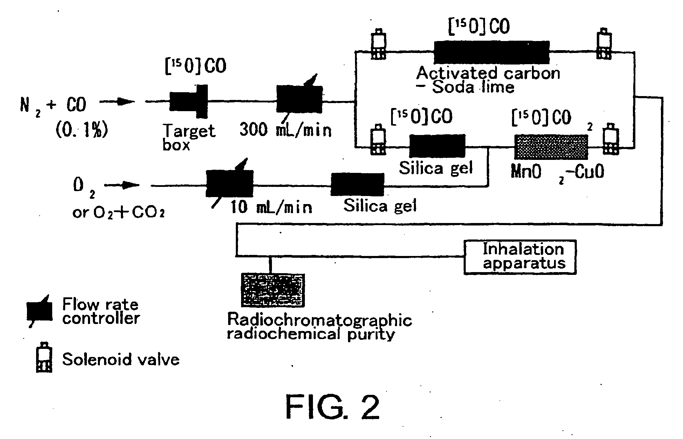

A system for producing [¹⁵O]CO and measuring radiochemical purity is shown in Fig. 2. As the gas to be irradiated, a high-purity nitrogen gas (99.9999 or more) comprising 0.1% carbon monoxide was flowed continuously into a target box at a flow rate of 300 mL/min. The target box was continuously irradiated with deuterons to cause the nuclear reaction of ¹⁴N (d,n)¹⁵O. The out-flowing reaction product was purified without heating, by passing through a column filled with activated carbon and soda lime. The [¹⁵O]CO comprised in the gas to be irradiated after purification was analyzed by radio-chromatography to reveal radiochemical purity of 99% or more.

### [EXAMPLE 2]

The gas to be irradiated comprising [¹⁵O]CO, obtained under the same conditions as in Example 1, was passed through a column filled with silica gel and activated carbon. Subsequently, dry oxygen (10 mL/min) dried with silica gel and a gas mixture (10 mL/min) of dry oxygen with 50 v/v% of dry carbon dioxide were added to the gas to be irradiated. Further, the mixture was passed through a manganese dioxide-copper oxide (II) catalyst. As the manganese dioxide-copper oxide (II) catalyst, CARULITE^{®} Catalyst (manufactured by STREM Chemicals, INC (Newburyport, MA, USA)) was used.

[¹⁵O]CO₂ comprised in the gas to be irradiated after oxidation by a catalyst was analyzed by radio-chromatography to reveal radiochemical purity of 96% or more. In particular, when a gas mixture (10 mL/min) comprising 50 v/v% of dry carbon dioxide was used, radiochemical purity increased to 99 v/v% or more.

### Industrial Applicability

The present invention provides methods for synthesizing [¹⁵O]CO or [¹⁵O]CO₂ that do not require a heating process. ¹⁵O gas could already be synthesized without a heating process. Therefore, the present invention makes it possible to supply all three of the gases necessary for a cerebral oxygen metabolism examination based on PET, without a heating process.

PET examination of cerebral oxygen metabolism provides important information for determining initial therapeutic strategies for cerebral stroke attacks. However, examination based on known methods takes two hours. The heating process in the synthesis of tracer gases occupies most of the examination time, becoming a significant cause of impeded reduction of examination time. The present invention realizes a substantial reduction in examination time, since synthesis of all tracer gases can be performed without heating process.

For example, [¹⁵O]CO gas is synthesized using the apparatus for synthesizing a tracer gas as described in the Examples. The gas to be irradiated, necessary for production of the [¹⁵O]CO gas, can be completely prepared in one minute. Since the present invention does not require a heating process, tracer gas synthesis can be immediately initiated if a cyclotron for deuteron irradiation is prepared. In contrast, known methods cannot synthesize tracer gases until heating is complete. The heating process requires around 30 minutes.

The following example (Table 1) shows the time required for a method of the present invention (low-temperature rapid synthesis method) compared with conventional methods, which require 26 minutes for heating. The method of the present invention saves 25 (26-1) minutes. That is, according to the present invention, the time required to examine cerebral oxygen metabolism, which is known to be about two hours, can be reduced by 20% or more.

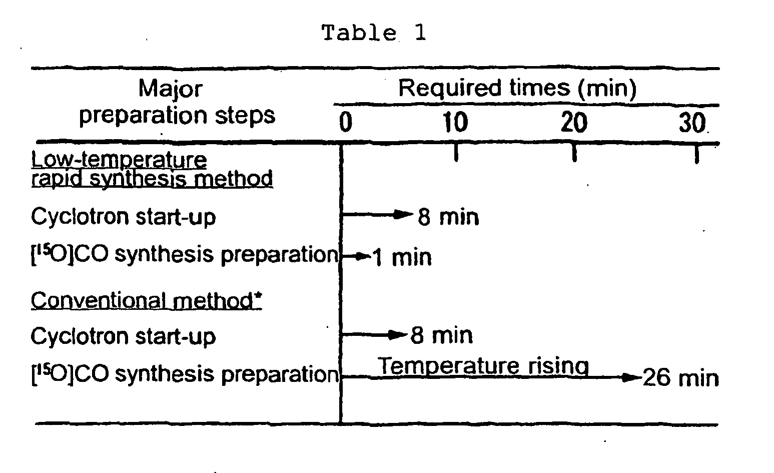

## Claims

1. A method for producing ¹⁵O-carbon monoxide at room temperature, which comprises the steps of:
(1) using a nitrogen gas comprising carbon monoxide as a gas to be irradiated, and irradiating the gas with a deuteron beam; and
(2) recovering ¹⁵O-carbon monoxide produced by the deuteron beam irradiation.

2. The method according to Claim 1, wherein the amount of carbon monoxide comprised in the gas to be irradiated is 0.01 to 0.5 v/v%.

3. The method according to Claim 2, wherein the amount of carbon monoxide comprised in the gas to be irradiated is 0.05 to 0.2 v/v%.

4. The method according to Claim 1, which comprises the step of contacting the irradiated gas with an active carbon column and/or a soda lime column, and recovering ¹⁵O-carbon monoxide.

5. The method according to Claim 1, wherein the gas to be irradiated is continuously supplied while recovering the produced ¹⁵O-carbon monoxide.

6. An apparatus for producing ¹⁵O-carbon monoxide at room temperature, which comprises:
(1) a target box through which a gas to be irradiated can be passed and in which the gas to be irradiated can be irradiated with a deuteron beam;
(2) a means of supplying the gas to be irradiated, which supplies the target box of (1) with a nitrogen gas comprising carbon monoxide as the gas to be irradiated; and
(3) a means for recovering ¹⁵O-carbon monoxide comprising an active carbon column and/or a soda lime column.

7. The apparatus according to Claim 6, wherein the means for supplying the gas to be irradiated of (2) can continuously supply the target box of (1) with the gas to be irradiated.

8. The apparatus according to Claim 6, wherein the means of (3) for recovering ¹⁵O-carbon monoxide is connected to a line for supplying ¹⁵O-carbon monoxide to a subject.

9. A method for producing ¹⁵O-carbon dioxide at room temperature, which comprises the steps of:
(1) contacting ¹⁵O-carbon monoxide produced by the method of claim 1 with a manganese dioxide-copper oxide (II) catalyst in the presence of dry oxygen; and
(2) recovering the produced ¹⁵O-carbon dioxide.

10. The method according to Claim 9, wherein the dry oxygen is a mixed gas comprising dry carbon dioxide.

11. An apparatus for producing ¹⁵O-carbondioxide at room temperature, which comprises:
(1) a means for supplying ¹⁵O-carbon monoxide wherein the means of (1) for supplying ¹⁵O-carbon monoxide is a ¹⁵O-carbon monoxide-producing apparatus that comprises:
i) a target box through which a gas to be irradiated can be passed, and in which the gas to be irradiated can be irradiated with a deuteron beam; and
ii) a means for supplying the gas to be irradiated which supplies the target box of (i) with a nitrogen gas comprising carbon monoxide as the gas to be irradiated;
(2) a manganese dioxide-copper oxide (II) catalyst;
(3) a means for supplying dry oxygen to the manganese dioxide-copper oxide (II) catalyst; and
(4) a means for recovering ¹⁵O-carbon dioxide.

12. A method for producing ¹⁵O-carbon monoxide and ¹⁵O-carbon dioxide at room temperature, which comprises the steps of:
(1) using a nitrogen gas comprising carbon monoxide as a gas to be irradiated, and irradiating the gas with a deuteron beam;
(2) recovering the ¹⁵O-carbon monoxide produced by the deuteron beam irradiation;
(3) contacting the irradiated gas with a manganese dioxide-copper oxide (II) catalyst in the presence of dry oxygen; and
(4) recovering ¹⁵O-carbon dioxide produced in the step of (3).

13. An apparatus for producing ¹⁵O-carbon monoxide and ¹⁵O-carbon dioxide at room temperature, which comprises:
(1) a target box through which a gas to be irradiated can be passed, and in which the gas to be irradiated can be irradiated with a deuteron beam;
(2) a means for supplying the gas to be irradiated, which supplies the target box with a nitrogen gas comprising carbon monoxide as the gas to be irradiated;
(3) a means for recovering ¹⁵O-carbon monoxide;
(4) a manganese dioxide-copper oxide (II) catalyst for oxidizing carbon monoxide;
(5) a means for supplying dry oxygen to the oxidizing catalyst; and
(6) a means for recovering ¹⁵O-carbon dioxide.

## Patentansprüche

1. Verfahren zum Herstellen von ¹⁵O-Kohlenmonoxid bei Raumtemperatur, welches die Schritte umfasst:
(1) Verwenden eines Stickstoffgases, welches Kohlenmonoxid umfasst, als ein zu bestrahlendes Gas und Bestrahlen des Gases mit einem Deuteronenstrahl; und
(2) Gewinnen des durch die Deuteronenstrahlbestrahlung hergestellten ¹⁵O-Kohlenmonoxids.

2. Verfahren gemäß Anspruch 1, worin die Menge an Kohlenmonoxid, welche im zu bestrahlenden Gas umfasst ist, 0,01 bis 0,5 Vol./Vol.-% ist.

3. Verfahren gemäß Anspruch 2, worin die Menge an Kohlenmonoxid, welche im zu bestrahlenden Gas umfasst ist, 0,05 bis 0,2 Vol./Vol.-% ist.

4. Verfahren gemäß Anspruch 1, welches den Schritt des Kontaktierens des bestrahlten Gases mit einer Aktivkohle-Säule und/oder einer Natronkalk-Säule und Gewinnen von ¹⁵O-Kohlenmonoxid umfasst.

5. Verfahren gemäß Anspruch 1, worin das zu bestrahlende Gas kontinuierlich zugeführt wird, während das hergestellte ¹⁵O-Kohlenmonoxid gewonnen wird.

6. Apparat zum Herstellen von ¹⁵O-Kohlenmonoxid bei Raumtemperatur, welcher umfasst:
(1) einen Zielkasten, durch welchen ein zu bestrahlendes Gas passiert werden kann und in welchem das zu bestrahlende Gas mit einem Deuteronenstrahl bestrahlt werden kann;
(2) ein Mittel zum Zuführen des zu bestrahlenden Gases, welches den Zielkasten von (1) mit einem Stickstoffgas, welches Kohlenmonoxid umfasst, als zu bestrahlendes Gas speist; und
(3) ein Mittel zum Gewinnen von ¹⁵O-Kohlenmonoxid, welches eine Aktivkohle-Säule und/oder eine Natronkalk-Säule umfasst.

7. Apparat gemäß Anspruch 6, worin das Mittel zum Zuführen des zu bestrahlenden Gases von (2) den Zielkasten von (1) kontinuierlich mit dem zu bestrahlenden Gas speisen kann.

8. Apparat gemäß Anspruch 6, worin das Mittel von (3) zum Gewinnen von ¹⁵O-Kohlenmonoxid an eine Leitung zum Zuführen von ¹⁵O-Kohlenmonoxid zu einem Objekt angeschlossen ist.

9. Verfahren zum Herstellen von ¹⁵O-Kohlendioxid bei Raumtemperatur, welches die Schritte umfasst:
(1) Kontaktieren von ¹⁵O-Kohlenmonoxid, hergestellt durch das Verfahren von Anspruch 1, mit einem Mangandioxid-Kupferoxid (II) Katalysator in Gegenwart von trockenem Sauerstoff; und
(2) Gewinnen des hergestellten ¹⁵O-Kohlendioxids.

10. Verfahren gemäß Anspruch 9, worin der trockene Sauerstoff ein gemischtes Gas ist, welches trockenes Kohlendioxid umfasst.

11. Apparat zum Herstellen von ¹⁵O-Kohlendioxid bei Raumtemperatur, welcher umfasst:
(1) ein Mittel zum Zuführen von ¹⁵O-Kohlenmonoxid, wobei das Mittel von (1) zum Zuführen von ¹⁵O-Kohlenmonoxid ein ¹⁵O-Kohlenmonoxid-herstellender Apparat ist, der umfasst:
i) einen Zielkasten, durch welchen ein zu bestrahlendes Gas passiert werden kann, und in welchem das zu bestrahlende Gas mit einem Deuteronenstrahl bestrahlt werden kann; und
ii) ein Mittel zum Zuführen des zu bestrahlenden Gases, welches den Zielkasten von (i) mit einem Stickstoffgas, welches Kohlenmonoxid umfasst, als zu bestrahlendes Gas speist;
(2) einen Mangandioxid-Kupferoxid (II) Katalysator;
(3) ein Mittel zum Zuführen von trockenem Sauerstoff zum Mangandioxid-Kupferoxid (II) Katalysator; und
(4) ein Mittel zum Gewinnen von ¹⁵O-Kohlendioxid.

12. Verfahren zum Herstellen von ¹⁵O-Kohlenmonoxid und ¹⁵O-Kohlendioxid bei Raumtemperatur, welches die Schritte umfasst:
(1) Verwenden eines Stickstoffgases, welches Kohlenmonoxid umfasst, als zu bestrahlendes Gas, und Bestrahlen des Gases mit einem Deuteronenstrahl;
(2) Gewinnen des durch die Deuteronenstrahlbestrahlung hergestellten ¹⁵O-Kohlenmonoxids;
(3) Kontaktieren des bestrahlten Gases mit einem Mangandioxid-Kupferoxid (II) Katalysator in Gegenwart von trockenem Sauerstoff; und
(4) Gewinnen des im Schritt von (3) hergestellten ¹⁵O-Kohlendioxids.

13. Apparat zum Herstellen von ¹⁵O-Kohlenmonoxid und ¹⁵O-Kohlendioxid bei Raumtemperatur, welcher umfasst:
(1) einen Zielkasten, durch welchen ein zu bestrahlendes Gas passiert werden kann, und in welchem das zu bestrahlende Gas mit einem Deuteronenstrahl bestrahlt werden kann;
(2) ein Mittel zum Zuführen des zu bestrahlenden Gases, welches den Zielkasten mit einem Stickstoffgas, welches Kohlenmonoxid umfasst, als das zu bestrahlende Gas speist;
(3) ein Mittel zum Gewinnen von ¹⁵O-Kohlenmonoxid;
(4) einen Mangandioxid-Kupferoxid (II) Katalysator zum Oxidieren von Kohlenmonoxid;
(5) ein Mittel zum Zuführen von trockenem Sauerstoff zum oxidierenden Katalysator; und
(6) ein Mittel zum Gewinnen von ¹⁵O-Kohlendioxid.

## Revendications

1. Procédé de production de monoxyde de ¹⁵O-carbone à température ambiante, comprenant les étapes consistant à :
(1) utiliser un gaz azoté comprenant du monoxyde de carbone en tant que gaz à irradier et irradier le gaz avec un faisceau de deutéron ; et
(2) récupérer le monoxyde de ¹⁵O-carbone produit par l'irradiation avec le faisceau de deutéron.

2. Procédé selon la revendication 1, dans lequel la quantité de monoxyde de carbone comprise dans le gaz à irradier est de 0,01 à 0,5% en volume/volume.

3. Procédé selon la revendication 2, dans lequel la quantité de monoxyde de carbone comprise dans le gaz à irradier est de 0,05 à 0,2% en volume/volume.

4. Procédé selon la revendication 1, comprenant l'étape consistant à mettre en contact le gaz irradié avec une colonne de charbon actif et/ou une colonne de chaux sodée et à récupérer le monoxyde de ¹⁵O-carbone.

5. Procédé selon la revendication 1, dans lequel le gaz à irradier est fourni en continu tout en récupérant le monoxyde de ¹⁵O-carbone produit.

6. Appareil de production de monoxyde de ¹⁵O-carbone à température ambiante, comprenant :
(1) une boîte à cible à travers laquelle un gaz à irradier peut passer et dans laquelle le gaz à irradier peut être irradié avec un faisceau de deutéron ;
(2) un moyen d'alimentation en gaz à irradier qui alimente la boîte à cible de (1) en gaz azoté comprenant du monoxyde de carbone en tant que gaz à irradier ; et
(3) un moyen pour récupérer le monoxyde de ¹⁵O-carbone comprenant une colonne de charbon actif et/ou une colonne de chaux sodée.

7. Appareil selon la revendication 6, dans lequel le moyen d'alimentation en gaz à irradier de (2) peut alimenter en continu la boîte à cible de (1) en gaz à irradier.

8. Appareil selon la revendication 6, dans lequel le moyen de (3) pour récupérer le monoxyde de ¹⁵O-carbon est connecté à une conduite permettant de fournir le monoxyde de ¹⁵O-carbone à un sujet.

9. Procédé de production de dioxyde de ¹⁵O-carbone à température ambiante, comprenant les étapes consistant à :
(1) mettre en contact le monoxyde de ¹⁵O-carbone produit par le procédé de la revendication 1 avec un catalyseur à base de dioxyde de manganèse et d'oxyde de cuivre (II) en présence d'oxygène sec ; et
(2) récupérer le dioxyde de ¹⁵O-carbone produit.

10. Procédé selon la revendication 9, dans lequel l'oxygène sec est un gaz mélangé comprenant du dioxyde de carbone sec.

11. Appareil de production de dioxyde de ¹⁵O-carbone à température ambiante, comprenant :
(1) un moyen d'alimentation en monoxyde de ¹⁵O-carbone dans lequel le moyen de (1) pour alimenter en monoxyde de ¹⁵O-carbone est un appareil de production de monoxyde de ¹⁵O-carbone comprenant :
i) une boîte à cible à travers laquelle un gaz à irradier peut passer et dans laquelle le gaz à irradier peut être irradié avec un faisceau de deutéron ; et
ii) un moyen d'alimentation en gaz à irradier qui alimente la boîte à cible de (i) en gaz azoté comprenant du monoxyde de carbone en tant que gaz à irradier ;
(2) un catalyseur à base de dioxyde de manganèse et d'oxyde de cuivre (II) ;
(3) un moyen permettant de fournir de l'oxygène sec au catalyseur à base de dioxyde de manganèse et d'oxyde de cuivre (II) ; et
(4) un moyen de récupération du dioxyde de ¹⁵O-carbone.

12. Procédé de production de monoxyde de ¹⁵O-carbone et de dioxyde de ¹⁵O-carbone à température ambiante, comprenant les étapes consistant à :
(1) utiliser un gaz azoté comprenant du monoxyde de carbone en tant que gaz à irradier et irradier le gaz avec un faisceau de deutéron ;
(2) récupérer le monoxyde de ¹⁵O-carbone produit par l'irradiation avec le faisceau de deutéron ;
(3) mettre en contact le gaz irradié avec un catalyseur à base de dioxyde de manganèse et d'oxyde de cuivre (II) en présence d'oxygène sec ; et
(4) récupérer le dioxyde de ¹⁵O-carbone produit dans l'étape (3).

13. Appareil de production de monoxyde de ¹⁵O-carbone et de dioxyde de ¹⁵O-carbone à température ambiante, comprenant :
(1) une boîte à cible à travers laquelle un gaz à irradier peut passer et dans laquelle le gaz à irradier peut être irradié avec un faisceau de deutéron ;
(2) un moyen d'alimentation en gaz à irradier qui alimente la boîte à cible en gaz azoté comprenant du monoxyde de carbone en tant que gaz à irradier ;
(3) un moyen de récupération du monoxyde de ¹⁵O-carbone ;
(4) un catalyseur à base de dioxyde de manganèse et d'oxyde de cuivre (II) pour oxyder le monoxyde de carbone ;
(5) un moyen permettant de fournir de l'oxygène sec au catalyseur d'oxydation ; et
(6) un moyen de récupération du dioxyde de ¹⁵O-carbone .
